# EUROPEAN PATENT APPLICATION

(11) **EP 4 434 515 A1**
(43) Date of publication of application: **25.09.2024**
(21) Application number: 23382276.6
(22) Date of filing: 24.03.2023
(51) Int. Cl.: A61K 9/00, A61K 9/107, A61K 31/00, A61K 33/00, A61P 27/04, A61P 27/06, A61K 47/02, A61K 47/10, A61K 47/36

(54) **CBD FOR OCULAR USE**

(71) Applicant: Laboratoire Laredo S.L., 01012 Victoria-Gasteiz (Alava) (ES)
(72) Inventor: Moreno Martín, Ricardo Vidal, 01012 Vitoria-Gasteiz (ES); Nury, Chloé, 01012 Vitoria-Gasteiz (FR)
(74) Representative: Isern Patentes y Marcas S.L.

(57) **Abstract**

The invention provides an aqueous micellar solution comprising micelles of poloxamer, with a hydrophilic surface and a hydrophobic nucleus encapsulating CBD, which solution can additionally comprise NaCl, H₃BO₃, and sodium hyaluronate, and which is for use in the prevention and/or treatment of glaucoma, and/or high intraocular pressure, ocular dryness, ocular discomfort or pain and/or high mechanical ocular sensitivity. The administration of the micellar solution in drops to the eyes gives rise to a decrease in the intraocular pressure after repeated administration, preferably for seven days or more, without increasing mechanical corneal sensitivity or spontaneous tear production. The decrease in intraocular pressure should prevent or slow glaucoma appearance or development. Compositions for the same use, comprising the aqueous micellar solution, are also provided.

## Description

### FIELD OF THE INVENTION

The present invention belongs to the field of treatment of ocular diseases. More specifically, the invention relates to a formulation of cannabidiol (CBD) in the form of micelles and its use in the treatment of glaucoma and some associated symptoms, such as intraocular pressure, ocular dryness, ocular discomfort or pain and/or high mechanical ocular sensitivity.

### BACKGROUND OF THE INVENTION

Glaucoma represents a group of optic neuropathies of different aetiologies, essentially characterised by progressive retinal ganglion cell death and loss of optic nerve fibres, which subsequently leads to irreversible blindness. Glaucoma poses a significant public health concern as it is the second leading cause of blindness after cataracts, and this blindness is usually irreversible. It is estimated that 57.5 million people worldwide are affected by primary open-angle glaucoma (POAG), the most common type. People over 40-60 years of age, family members of those already diagnosed with glaucoma, steroid users, diabetics, as well as those with high myopia, hypertension, central cornea thickness of <5 mm, and eye injury are at an increased risk of glaucoma. By 2020, it is expected that approximately 76 million people will suffer from glaucoma with that number estimated to reach 111.8 million by 2040.

Elevated intraocular pressure (IOP) connects most forms of glaucoma. Previous reports (Heijl *et al.,* 2002; Gaudana *et al.,* 2010) indicate that IOP reduction can slow/prevent the progression of glaucoma. It is estimated that every mmHg reduction in IOP could entail about a 10% reduced risk of progression. However, as mentioned before the pathogenesis of glaucoma is complex and a strategy other than IOP reduction is required since clinical evidence suggests that current therapies can lessen but not halt the progression of the neuropathy.

Most treatments are focused exclusively on reducing intraocular pressure by pharmacological treatments: analogues of prostaglandins, β-adrenergic antagonists, cholinergic agonists, α2-adrenergic agonists, carbonic anhydrase inhibitors and rho-kinase inhibitors (Aebersold et al., 2021). But, additionally to ocular hypertension, corneal dryness and nociception are frequently reported causes of ocular morbidity. However, when one issue is singled out, its contribution to pathogenesis may not necessarily be critical. Moreover, given the multiple features in glaucoma disease (such as elevated IOP, oxidative disorder, immune dysfunction, vasoconstriction or congenital mutations), a specific treatment addressed to every single pathogenic mechanism may constitute an enormous task. In this regard, effective therapies should target pathways and processes that drive neurodegeneration and behavioural assessment in animal models covering distinct symptoms of the disease may constitute optimal predictive tools.

Phytocannabinoids are not currently considered a suitable first-line therapeutic for glaucoma (e.g., American Academy of Ophthalmology [AAO] position statement 2014, www.aao.org); however, this may be based on limited evidence. The central argument is that topical THC is not effective, therefore necessitating treatment via cannabis inhalation. Cannabis inhalation, in turn, has assorted shortcomings: (1) psychoactivity, (2) short action (<4 hours), and (3) elevation of blood pressure. A key question then is whether topical THC works in humans (Miller et al., 2018).

In order to design effective therapies, it must be taken into account that one of the biggest challenges in ophthalmic treatment is that drugs must be highly liposoluble to cross the five membranes that make up the cornea, but water-soluble enough to dissolve in the tear film that is on top of it and that prevents direct contact with the ocular surface. Additionally, its lipophilicity makes its topical delivery to the back of the eye challenging (Taskar et al., 2019)95% of the medication applied directly to the eye leaves it in the first few seconds without giving it time to reach the intraocular tissues or the back of the eye, which are reached by less than 5% of the applied doses. The medication must also be non-irritating, sterile and, as far as possible, not interfere with vision.

Micelles, among others, have been one of the nanoparticles studied in the targeting of drug substances to different tissues and organs (such as the eye, for instance) as a nano-sized delivery system for many years. Micelles encapsulate drugs into a lipophilic core with an outside layer of hydrophilic heads. Micelles are often used in the form of micellar solutions. A micellar solution consists of a dispersion of micelles in a solvent (most usually water). Micelles consist of aggregated amphiphiles, and in a micellar solution these are in equilibrium with free, unaggregated amphiphiles. An amphiphile, in turn, is a chemical compound possessing both hydrophilic and lipophilic properties; soaps, detergents and lipoproteins are common amphiphilic substances. Micellar solutions form when the concentration of amphiphile exceeds the critical micellar concentration (CMC) or critical aggregation concentration - CAC, and persist until the amphiphile concentration becomes sufficiently high to form a lyotropic liquid crystal phase (Oh S.G. et al., 2007).

Among the amphiphilic compounds that have been extensively used to form micelles for use in the cosmetic and therapeutic fields are poloxamers. For instance, Wang and his group recently developed a thermosensitive nanogel of muscone (a poorly water soluble drug) by the reverse micelle/positive micelle (RM-PM) method using poloxamers (Wang G. et al., 2016) . Poloxamers are hydrophilic non-ionic surfactants of the class of copolymers. Specifically, poloxamers are triblock polymers consisting of a central hydrophobic block of polypropylene glycol (which blocks can be also referred as PPO, an abbreviation from the alternative denomination (poly)propylene oxide), flanked by two hydrophilic blocks of polyethylene glycol (PEG) (also abbreviated as PEO, from the alternative nomenclature (poly)ethylene oxide). The length of the polymer blocks can be customized, so that different poloxamers exist with not very different properties, Poloxamer 407 being one of most used ones. This particular compound is also known by some of the trade names under which it is commercialised: Synperonic PE/F-127 (Croda), Pluronic F-127 and its pharmaceutical grade version Kolliphor P (both of BASF).

Other different amphiphilic compounds are used to form micelles for being used in the pharmaceutical field.

As reviewed, for instance, by Durgun *et al.* in 2020, sustained drug release, ease of production, increased solubility, and bioavailability of drugs with low water solubility are the most important superiority of micellar carriers. These advantages paved the way for the use of micelles as a drug delivery system in the ocular tissues. But the eye anatomical structure entails that conventional dosage forms can only reach the anterior segment of the eye, so that micelles are mostly used for the treatment of diseases of this segment. In the treatment of posterior segment diseases, conventional dosage forms are administered sclerally, via an intravitreal injection, or systemically; otherwise, dosage forms cannot reach eye posterior/intraocular tissue The potential of micellar systems ocular drug delivery has been demonstrated by *in vivo* animal experiments and clinical studies performed using different administration routes, although the feasibility of topical administration to deliver drugs to the eye posterior segment is still not well demonstrated.

One of the compounds for which micelles have been used as vehicles, mainly for enhancing oral bioavailability in anti-inflammatory therapy (Rao *et al.,* 2022) or facilitating is topical administration to the skin (see, for instance, US patent application published as US20200108014A1), is cannabidiol (CBD). CBD is one of the cannabinoids identified in cannabis (*Cannabis sativa*) plants. Its chemical formula is.

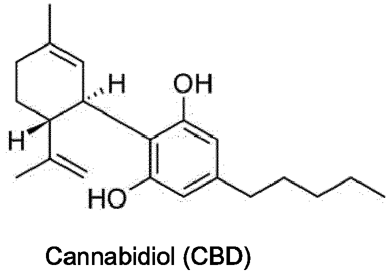

It lacks psychotropic effects, but has been associated with several therapeutic effects. CBD has been shown to act on nerves, having antiepileptic activity (Schaiquevich *et al.,* 2020), as well as exhibit analgesic and anti-inflammatory properties in basic research studies, which properties could potentially help to treat inflammation and pain (Taskar et al., 2019). In the United States, a medicament containing CBD as active principle has been approved for the FDA (Food and Drug Administration) for the treatment of epileptic seizures associated with Dravet syndrome, Lennox-Gastaut syndrome or tuberous sclerosis complex, while in the European Union the same medicament is indicated for use as adjunctive therapy of seizures associated with Lennox Gastaut syndrome (LGS) or Dravet syndrome (DS), in conjunction with clobazam. In Spain and other European countries, the only approved medicament with cannabis extracts is commercialised under the denomination Sativex^{®} and is the form of an oromucosal spray made from the whole cannabis plant, containing the cannabinoid Δ⁹-tetrahydrocannabinol (THC) additionally to cannabidiol at a 1:1 approximate rate, which is indicated for moderate or severe forms of spasticity in patients with multiple sclerosis. CBD is also a popular herbal dietary supplement, sold in parapharmacies and herbalists as oils, creams, balsams, patches... and even as edible products in some countries, sometimes claiming particular therapeutic effects that lack scientific evidence or are not completely proven (Iffland *et al.,* 2017). Its use for topical administration to the skin, in micellar form, has been also proposed, as can be seen in US patent application published as US20200108014A1.

CBD has been also tested as an ocular therapeutic agent, as a recent review by Aebersold and others discusses (Aebersold *et al.,* 2021) . Clinical trials in patients with high intraocular pressure have shown discordant results regarding its efficacy after CBD sublingual administration (Tomida *et al.,* 2006). Discordant results have also been reported in animal assays carried out in the Indiana University (Miller *et al.,* 2018):Such results appear to indicate that acute topical CBD increases IOP and, in general, they have been interpreted as suggesting that the use of the substance in the treatment of glaucoma may actually worsen the condition. The article where the assays were published was cited by the American academy of ophthalmology with the headline "CBD oil may worsen glaucoma" (Shelton, 2019). Altogether, the literature does not conclusively show whether CBD increases, decreases or causes no change to IOP. It should be also commented that no study with chronic CBD administration to the eye has been published.

Additionally, CBD lipophilicity makes its topical delivery to the back of the eye challenging. As discussed in the above mentioned review by Aebersold and others, therapeutic treatments for ocular conditions are frequently administered orally or topically to the eye. Extensive first pass metabolism of CBD prevents a significant amount of drug reaching the eye from oral administration. Therefore, topical administration of CBD is desired. But developing an ocular topical delivery system is a difficult task. The eye contains sophisticated protective mechanisms and physical barriers to prevent foreign material from entering, which includes the multi-layered cornea and precorneal tear film. The alternating lipophilic and hydrophilic nature of the cornea makes ocular drug delivery exceptionally difficult. As a result, less than 5% of drugs applied topically enter the eye.

CBD is highly hydrophobic and insoluble in water. Studies applying CBD topically used non-aqueous vehicles for delivery (Thapa *et al.,* 2018, Miller *et al.,* 2018; Green *et al.,* 1978). Previously, CBD was topically delivered to the eye in different oils: mineral oil, sesame oil, soybean oil, and a soya oil/water emulsion, Tocrisolve (Thapa *et al.,* 2018, Miller *et al.,* 2018; Green *et al.,* 1978). In one report, CBD delivered in mineral oil produced an IOP-lowering effect whilst the effect is absent in sesame oil (Green *et al.,* 1978). The soya oil/water emulsion was the used vehicle in the above mentioned study where Indiana University researchers (Miller *et al.,* 2018) demonstrated that a high dose of CBD increases IOP in wildtype mice, but decreases IOP in CB1 knockout mice. This indicates that at large doses CBD might produce off-target effects which are detrimental. Since CBD has at least 65 targets (Ibeas Bih *et al.,* 2015), off-target effects of CBD at high doses are very likely.

These results highlight a critical need for a vehicle with high ocular permeation to administer CBD topically in a therapeutically relevant dose, with the appropriate dosage regime, so that positive effects of CBD on reducing high IOP in human beings and other mammals can be observed sustainably, thus reducing the risk of glaucoma. But this issue remains unsolved.

Meanwhile, the incidence of glaucoma continues to increase in the population and the age of appearance is increasingly lower, due to the earlier use of devices with screens, which accelerates glaucoma generation.

Thus, it will be interesting to have a CBD delivery system that could allow its administration to the eye in an effective and controlled manner so that it could reduce IOP and, consequently, prevent or lessen glaucoma progression. Additionally, it would be preferable that CBD administered by that system would also ameliorate other glaucoma signs, such as pain.

The present invention provides a solution to such a problem.

### SUMMARY OF THE INVENTION

The present invention relates to an aqueous micellar solution comprising micelles formed by a poloxamer, characterised in that cannabidiol (CBD) is encapsulated in the micelles, for use in the prevention and/or treatment of glaucoma, elevated intraocular pressure, ocular dryness, ocular discomfort or pain and/or high mechanical eye sensitivity, wherein the solution is to be administered by the topical ophthalmic route.

In a preferred embodiment, the poloxamer is poloxamer 407.

In another preferred embodiment, compatible with the previous one, the relative proportion, weight: weight, poloxamer: CBD in the solution is 9 : 3 ± 0.30.

In another possible embodiment, the aqueous micellar solution for use according to the invention comprises at least

| | |
|---|---|
| Cannabidiol | 0.00282 - 0.00318 g/ml |
| Poloxamer | 0.00846 - 0.00954 g/ml |
| Type II Water | q.s.p. the desired volume. |

Such aqueous micellar solution would be the solution for being administered as drops, by the topical ophthalmic route. The concentrations are calculated taking into account the total amount of each ingredient in the solution and based on the total volume of the solution, including micelles and vehicle.

Preferably, the above micellar solution for being administered as drops additionally comprises

| | |
|---|---|
| NaCl | 0.141 - 0.159 mg/ml |
| H₃BO₃ | 14.10 - 15.90 mg/ml |
| Sodium hyaluronate | 1.175 - 1.325 mg/ml |
| Type II Water | q.s.p. the desired volume. |

Again, the concentrations are calculated taking into account the total amount of each ingredient in the solution and based on the total volume of the solution, including micelles and vehicle.

In a particular preferred embodiment of the previous one, combinable with any other embodiment, the micellar solution for use according to the invention (that is, for being administered as drops by the ophthalmic ocular route, for use in the prevention and/or treatment of glaucoma, elevated intraocular pressure, ocular dryness, ocular discomfort or pain and/or high mechanical eye sensitivity) comprises:

| | |
|---|---|
| Cannabidiol | 0.003 g/ml |
| Poloxamer 407 | 0.009 g/ml |
| NaCl | 0.15 mg/ml |
| H₃BO₃ | 15 mg/ml |
| Sodium hyaluronate | 1.25 mg/ml |
| Type II Water | q.s.p. the desired final volume. |

Again, the concentrations are calculated taking into account the total amount of each ingredient in the solution and based on the total volume of the solution, including micelles and vehicle.

In another possible embodiment, compatible with the previous ones, the micellar solution is for being administered at least for more than one day. Preferably, it is for repeated administration for more than four days, more preferably for more than seven days.

Another aspect of the invention is a composition comprising the micellar solution for use according to the present invention. In a possible embodiment the composition additionally comprises one or more pharmaceutically acceptable excipient(s). In another possible embodiment, compatible with the previous one, the composition also comprises one or more compound(s) selected from the group of: a prostaglandin analogue, a β-adrenergic antagonist, a cholinergic agonist, an α2-adrenergic agonist, a carbonic anhydrase inhibitor and a rho-kinase inhibitor.

### DESCRIPTION OF THE DRAWINGS

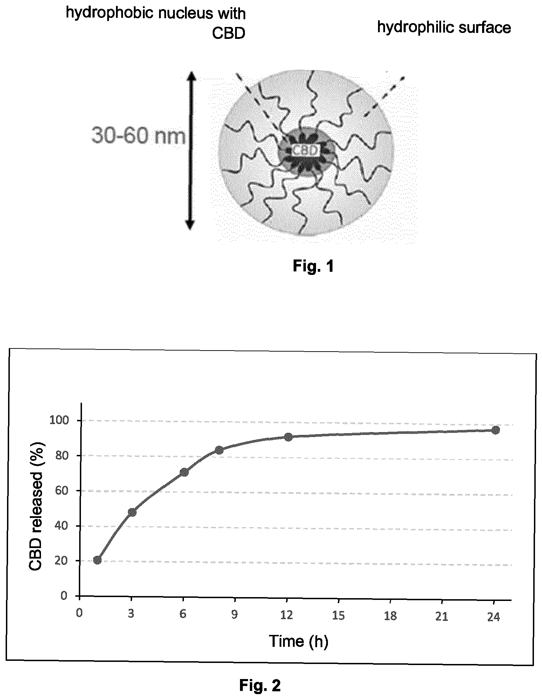

Fig. 1. Schematic representation of a micelle in accordance with the present invention.
Fig. 2. Representation of CBD release rate (percentage related to the total content in micelles) over time.

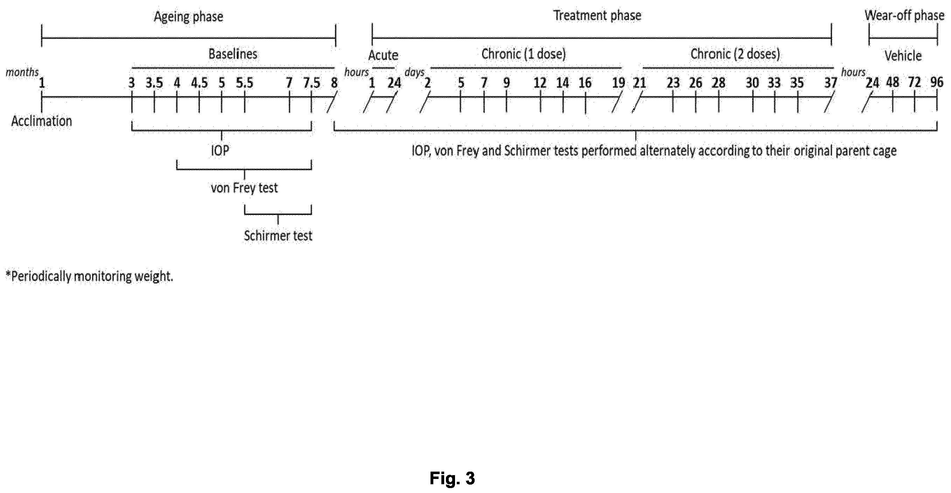

Fig. 3. Schematic representation of the performed experimental plan. IOP: intraocular pressure.

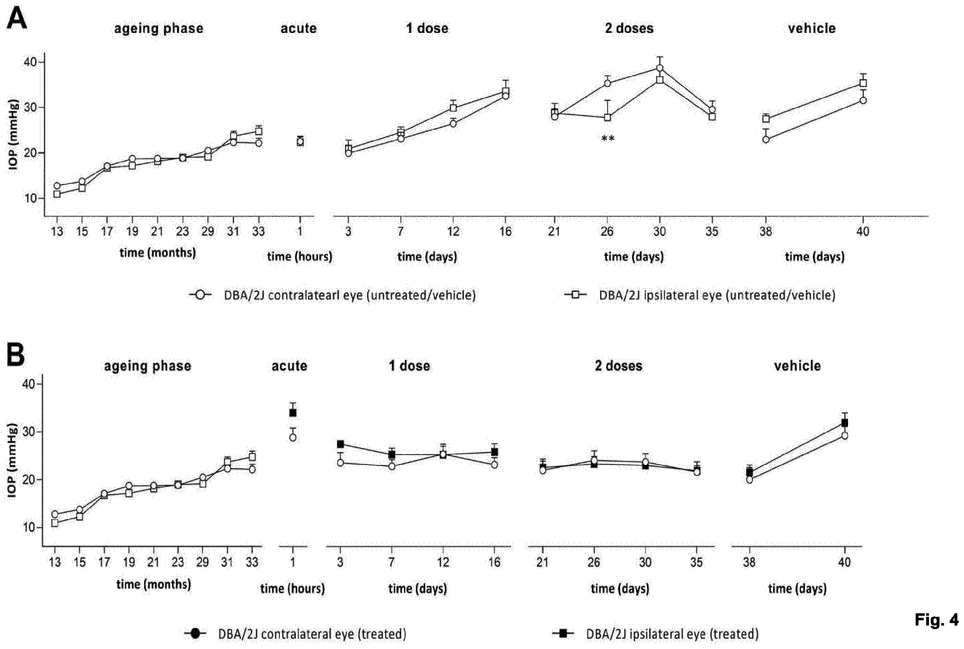

Fig. 4 contains line graphs of evolution of intraocular (IOP) pressure along time, each graph corresponding to one of the phases and subphases of the research program. Comparisons are made between contralateral (results represented by circles) and ipsilateral (results represented by squares) eyes, in untreated mice (panel A) and in treated mice (panel B).

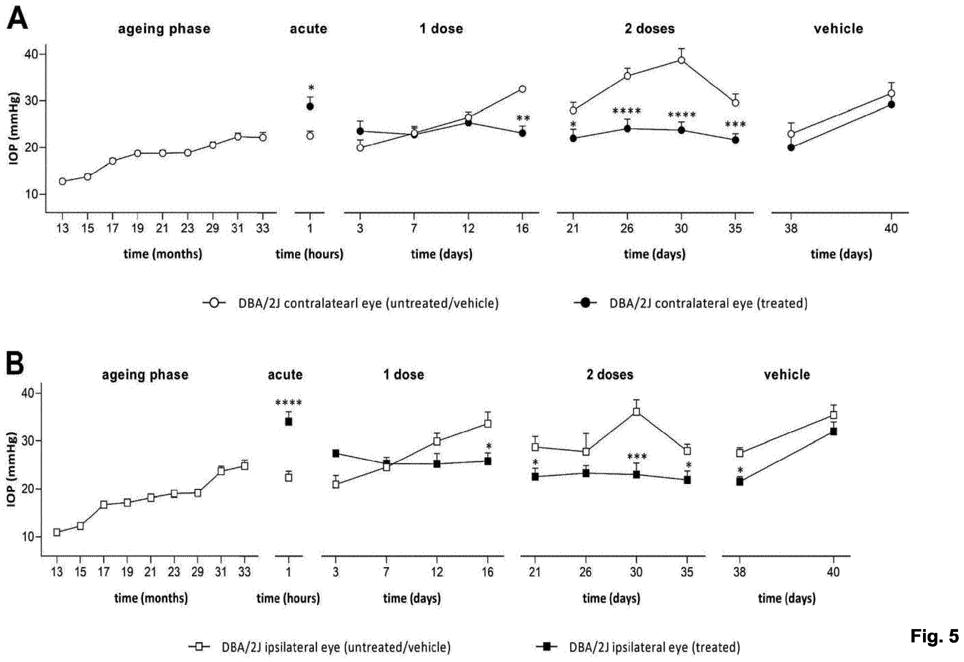

Fig. 5 contains line graphs of evolution of intraocular (IOP) pressure along time, each graph corresponding to one of the phases and subphases of the research program. Comparisons are made between untreated (results represented by empty symbols) and treated (results represented by filled symbols) mice, in contralateral eyes (panel A, results represented by circles) and in ipsilateral eyes (panel B, results represented by squares). *P<0.05,** P<0.01, ***P<0.001. ****P<0.0001.

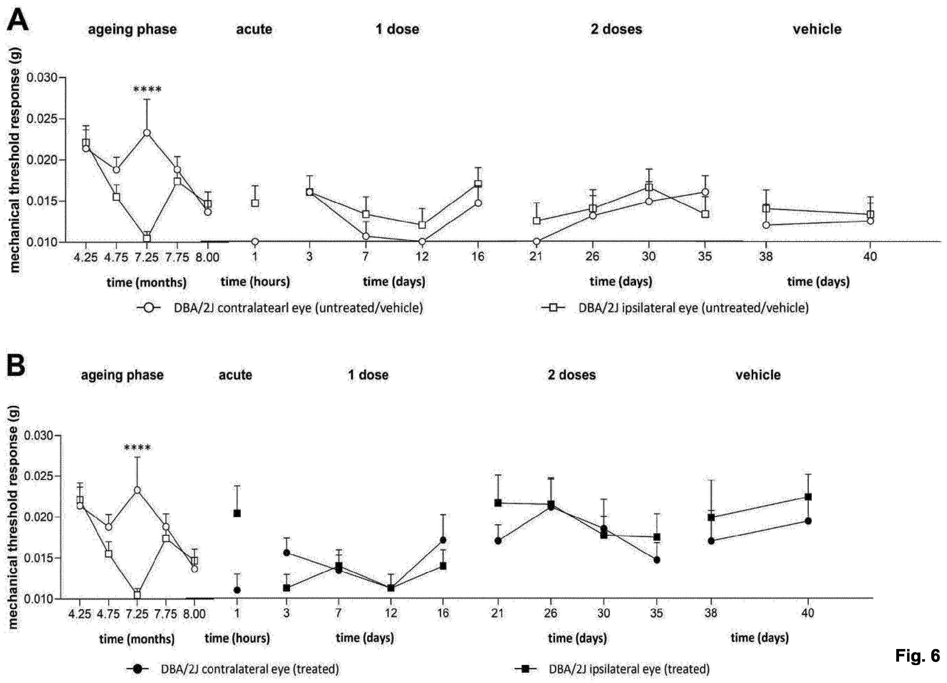

Fig. 6 contains line graphs of evolution of corneal mechanical sensitivity, paw withdrawal threshold (PWT: paw withdrawal threshold) in DBA/2J mice over a long time, each graph corresponding to one of the phases and subphases of the research program. Comparisons are made between contralateral (results represented by circles) and ipsilateral (results represented by squares) eyes, in untreated mice (panel A) and in treated mice (panel B).

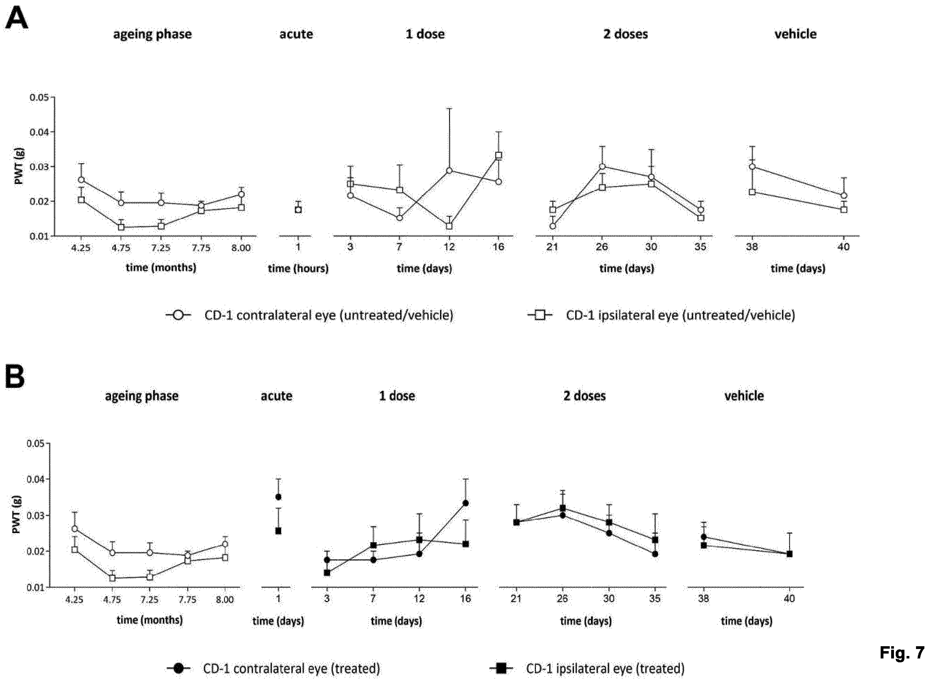

Fig. 7 contains line graphs of evolution of corneal mechanical sensitivity, (PWT: paw withdrawal threshold) in CD-1 mice over time, each graph corresponding to one of the phases and subphases of the research program. Comparisons are made between contralateral (results represented by circles) and ipsilateral (results represented by squares) eyes, in untreated mice (panel A) and in treated mice (panel B).

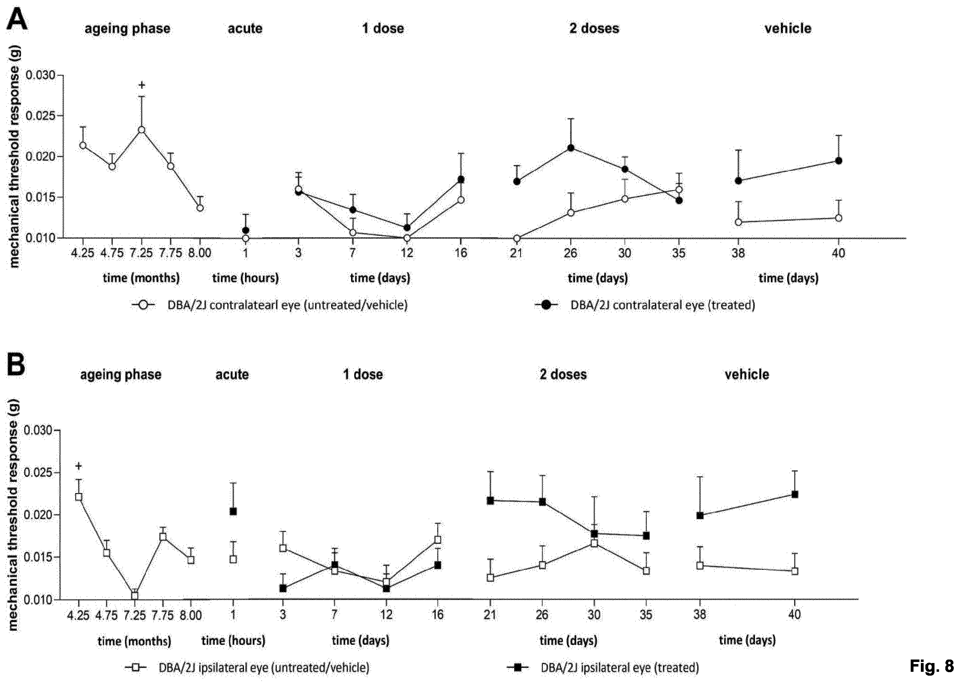

Fig. 8 contains line graphs of evolution of corneal mechanical sensitivity, (PWT: paw withdrawal threshold), in DBA/2J mice over time, each graph corresponding to one of the phases and subphases of the research program. Comparisons are made between untreated (results represented by empty symbols) and treated (results represented by filled symbols) mice, in contralateral eyes (panel A, results represented by circles) and in ipsilateral eyes (panel B, results represented by squares).

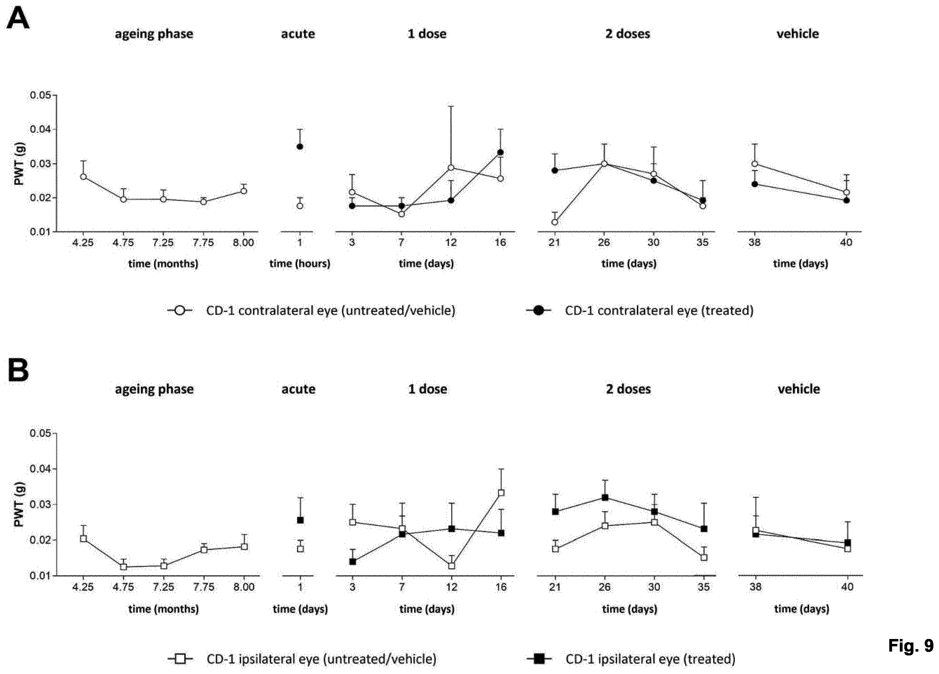

Fig. 9 contains line graphs of evolution of corneal mechanical sensitivity, (PWT: paw withdrawal threshold), in CD-1 mice over time, each graph corresponding to one of the phases and subphases of the research program. Comparisons are made between untreated (results represented by empty symbols) and treated (results represented by filled symbols) mice, in contralateral eyes (panel A, results represented by circles) and in ipsilateral eyes (panel B, results represented by squares).

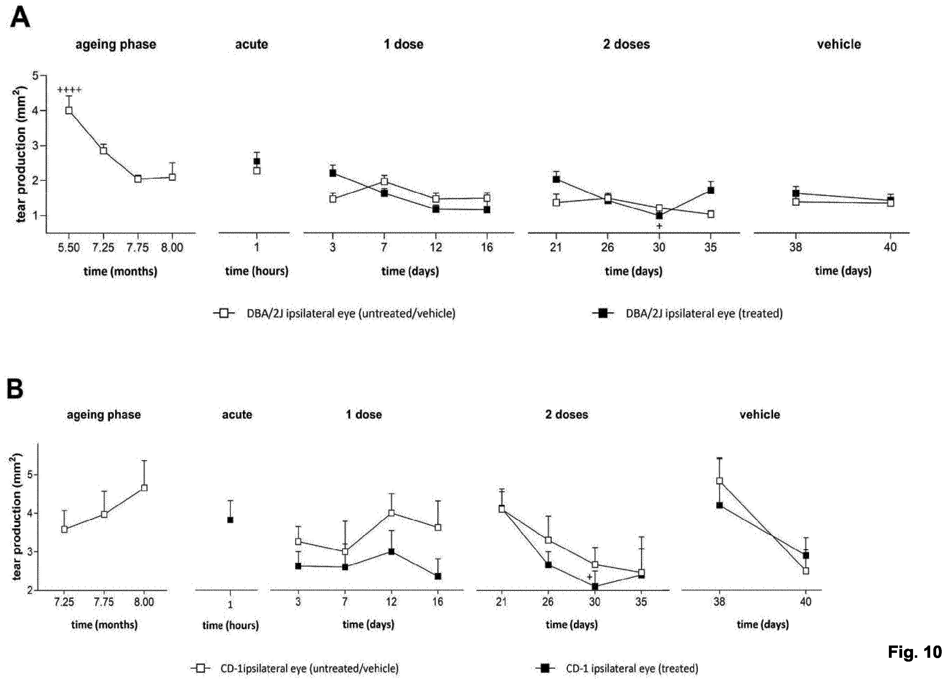

Fig. 10 contains line graphs of evolution of spontaneous production of tears, in ipsilateral eyes of either DBA/2J mice (panel A) or CD-1 mice (panel B) over time, each graph corresponds to one of the phases and subphases of the research program. Comparisons are made between untreated (results represented by empty symbols) and treated (results represented by filled symbols) mice.

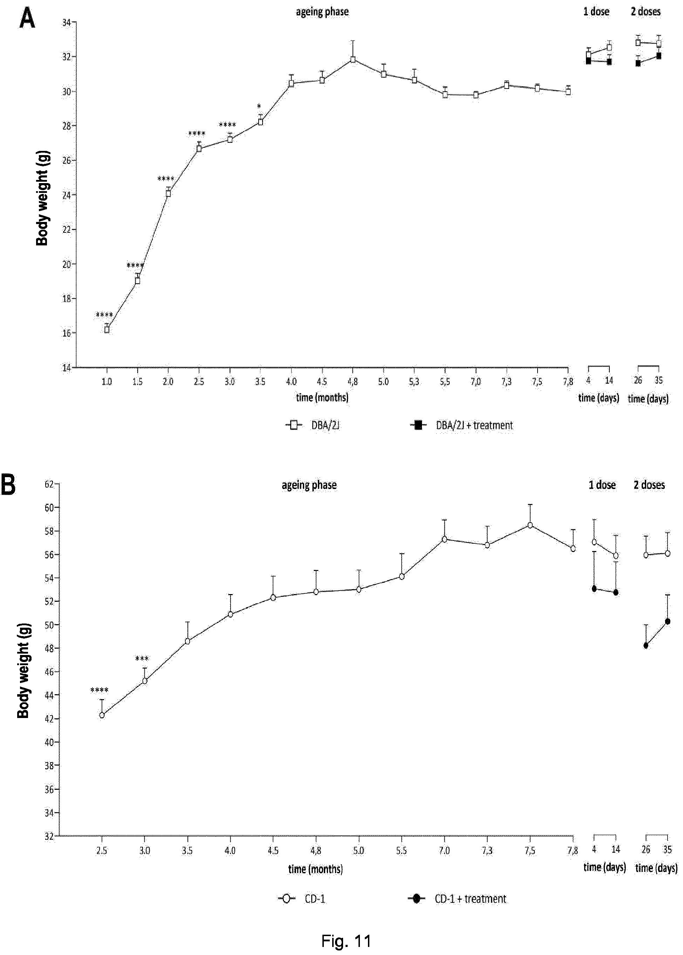

Fig. 11 contains line graphs of evolution of body weight, in DBA/2J mice (panel A) or CD-1 mice (panel B) over time, each graph corresponds to one of the phases and subphases of the research program. In the time periods where mice received CBD, comparisons are made between untreated (results represented by empty symbols) and treated (results represented by filled symbols) mice. *P<0.05, ***P<0.001, ****P<0.0001.

### DETAILED DESCRIPTION OF THE INVENTION

The present invention is based in the results of a study aimed, firstly, to study the analgesic efficacy and the effect of a hydrophilic CBD formulation on the intraocular pressure and ocular dryness in a model of glaucoma in mice, and, secondly, to examine the influence of the strain on the behavioural features aforementioned under acute and chronic CBD treatment, in order to assess the formulation utility for the treatment of glaucoma and/or some of its associated symptoms (intraocular pressure, ocular dryness, pain or discomfort and/or high mechanical eye sensitivity) in human beings and other mammals.

The assays carried out, described in the Examples below, revealed that the solution of the present invention, which comprises micelles wherein CBD is encapsulated, is able to reduce intraocular pressure by -70% compared to untreated animals and raise sensitivity thresholds by -50%, thus preventing discomfort/pain due to mechanical stimulation of the cornea.

The ophthalmic formulation used in the assays disclosed in the present application is a micellar hydrophilic ophthalmic solution designed to facilitate CBD topical administration to the eyes as drops in an optimal way. The solution has been designed taken into account both the lipophilic nature of CBD and the particular requirements for drugs and pharmaceutical compositions used for ophthalmic treatment related to eye structure and composition, particularly for those targeted to the eye posterior segment, that is: drugs and/or pharmaceutical compositions must be highly liposoluble in order to go through the five membranes that form the cornea but hydrophilic enough to be solved in the tear film that prevents direct contact with the ocular surface; as drugs and other substances and particles are drained by the lacrimal towards the posterior part of the nose for their elimination, if such requirements are not met, it is possible that a drug and/or pharmaceutical composition directly applied onto the eye abandons the eye within the first seconds after administration without having reached the intraocular tissues of the posterior part of the eye.

The solution provided by the present invention is a micellar solution (that is, a solution comprising micelles), or a composition which comprises said micellar solution, prepared by mixing a poloxamer with CBD in an aqueous medium, so that micelles are formed where the hydrophilic part of the molecules is in the micelle surface and where CBD is encapsulated in the hydrophobic core. Such micelles are for ocular treatment. They are schematically represented in Fig. 1, where it can be seen that they exhibit a hydrophilic surface and comprise a hydrophobic core (nucleus) where the CBD is encapsulated. The micelles have an average diameter of 30 - 60 nm.

Such micelles comprise aggregated molecules of an amphiphilic polymer, a poloxamer (which is poloxamer 407 in preferred embodiments of the invention), as well as encapsulated CBD. In an aqueous solution, when its critical micelle concentration (CMC) is reached, poloxamers aggregate forming micelles with a hydrophilic surface and a hydrophobic core. It should be noted that both temperature and pH are two important parameters to control since they are determining factors for the correct formation of these micelles. In the case of temperature, the spontaneous formation of micelles is determined by the temperature. For instance, in the particular case of the poloxamer used in the Examples of the present application, poloxamer 407, micelles can be formed within a range of 25-70°C, so that, at 25°C, approximately 20% of poloxamer in the form of micelles; outside of this range the micelles become solubilized. Moreover, it must be taken into account that the CMC decreases significantly as the temperature increases. Thus, if a poloxamer is added to an aqueous solution where CBD is present, when the poloxamer concentration reaches the CMC value, micelles are formed where CBD is encapsulated in a hydrophobic core which is compatible with the lipophilic nature of CBD.

Although other amphiphilic compounds could be used to obtain micelles in the aqueous solution, the use of poloxamers is preferred, since it has been demonstrated previously that gel formulations including 20-30% poloxamer exhibit no ocular irritation and toxicity (Mandal *et al.,* 2018). Among the poloxamers, poloxamer 407 is preferred for ophthalmic purposes like the one of the present invention.

The relative proportion of the poloxamer and CBD mixed for preparing the micelles, expressed as a weight proportion, can be 9 : 3±0.30, as in the case of Example 1, where the CBD encapsulation efficacy is 90% +/- 5% (a value of at least 85% being desirable).

The solution should be suitable for topical ocular administration. Thus, the solution should also exhibit the characteristics of the following Table:

**Table 1: Characteristics of the aqueous micellar solution**

| **Characteristic** | **Value** |
|---|---|
| Size of the micelles (average diameter) | 30 - 60 nm |
| Viscosity | 10 - 40 cp |
| pH | 6.8 - 7.8 (more preferably 7.2 - 7.6) |
| Osmolality | 200 - 400 mOsmol/kg |
| Visual appearance | Transparent homogenous liquid, without suspended particles or air bubbles |
| Sterile, non cytotoxic, biocompatible | |

Preferably, the micelles of the solution are in an aqueous vehicle, which comprises additional ingredients, which help to obtain a final solution with the desired properties in order to be administered to the eye by the topical ophthalmic route. In the present invention, the aqueous vehicle comprises at least sodium chloride (hypotonic), boric acid (as buffer forming compound for pH maintenance) and sodium hyaluronate (as rheological modifier and humectant), and water (preferably type II water) up to the desired final volume. The solution with the final formulation can be obtained by adding an initial micellar solution formed with poloxamer and cannabidiol to another solution comprising the selected additional ingredients. Preferably, for the purposes of the present invention, the final concentrations of the ingredients, based on the total mount of each ingredient in the solution (irrespective of being or not forming part of a micelle) and the final volume of the ophthalmic solution to be administered as drops by the topical ophthalmic route, are:

| | |
|---|---|
| Cannabidiol | 0.00282 - 0.00318 g/ml |
| NaCl | 0.1410 - 0.1590 mg/ml |
| H₃BO₃ | 14.10-15.90 mg/ml |
| Sodium hyaluronate | 1.175 - 1.325 mg/ml |
| Poloxamer 407 | 0.00846-0.00954 g/ml |
| Type II water | q.s.p. the desired final volume |

Regarding pH, it must be within a range of 4-8, more preferably 6.8-7.8 and even more preferably 7.2-7.6, to prevent degradation, so that it is advisable to prepare the micelles in a buffered solution. That is the reason why it is preferred in the present invention to use boric acid (H₃BO₃) as a buffer former at controlled pH..

Micelle formation, stability and suitable properties for ocular administration are also favoured by the additional ingredients: NaCl (sodium chloride) acts as hypotonic agent and sodium hyaluronate ((C₁₄H₂₁Na NO₁₁)ₙ) acts as rheological modifier and humectant. Together with boric acid, as mentioned above, their presence in the composition is preferred.

For the purposes of the present invention, it is possible to use sodium hyaluronate of different average molecular weights. For instance, it is possible to use high molecular weight sodium hyaluronate (HMW HA: 2000 - 2200 kDa) or low molecular weight sodium hyaluronate (LMW HA: 500 - 750 kDa), as in the Examples of the present application.

The aqueous micellar solution comprising encapsulated CBD fulfils the necessary requirements for being administered to the eye topically, as ophthalmic drops, for the treatment or prevention of alterations, dysfunctions or conditions of the posterior segment, since the aqueous nature of the solution, the hydrophilic nature of the micelle surface and the small size of the micelles facilitates both obtaining a sterile solution suitable for ophthalmic administration (by filtration through filters which do not retain the micelles, such as those of pore diameter near 0.2 micrometres, in aseptic conditions) and also the interaction with and dissolution in the lacrimal film of the eye surface, helping to lessen and/or slow the particle drainage to the nasal posterior part but facilitating CBD arrival to the posterior part of the eye and its contact with the corneal surface. Moreover, it is admitted that CBD liposolubility, in turn, facilitates its crossing of the corneal membranes and its arrival to the intraocular tissues. Moreover, encapsulation of CBD in micelles allows its controlled release, as shown in Example 1 below. Moreover, the solution does not contain any lumps or any undesired bodies and is also transparent, sterile, biocompatible, non-irritant and non-cytotoxic and, moreover, thanks to being in the form of a micellar solution, it does not provoke the sensation of discomfort that would provoke other particles that could be envisioned as possible vehicles for CBD such as, for instance, nanoparticles of mesoporous silica, that could be perceived as unpleasant foreign bodies by the patients. Therefore, the solution of the present invention exhibits important advantages for the direct administration to the eye, the administration route which is known as topical route or ophthalmic route in the ophthalmic field.

This approach differs from previous approaches where CBD administration encapsulated in micelles has been proposed, among others features, in the administration route, the organ where CBD effects is desired and/or the condition, dysfunction or alteration to treat.

For instance, in other published studies (such of that by Rao *et al.* in 2022), CBD is also administered encapsulated in micelles of poloxamer, but it is administered orally, micelles are used to increase bioavailability of CBD when administered by such route and the desired therapeutic effect is an anti-inflammatory effect.

In other studies, as discussed above (Thapa *et al.,* 2018; Miller *et al.,* 2018; Green et al., 1978), micelles had been assayed for CBD ocular administration, but in different oils, not obtaining effects in some cases (Green *et al.,* 1978) or observing IOP increases at CBD high doses in wild-type mice. The micelles designed for the purpose of the present invention, designed to mix themselves well with the lacrimal film, have shown to be a good vehicle for CBD administered by the topical ophthalmic route, demonstrating the feasibility of the use of said route for preventing or treating glaucoma and/or IOP by CBD administration, without increasing corneal sensitivity (and even decreasing it in the glaucoma animal model used) and without affecting severely welfare, as shown by the assays of body weight evolution.

In US patent application US20200108014A1, in turn, CBD administration in micelles is also proposed but contrary to the present invention, neither poloxamers nor any other block copolymer are mentioned as possible amphiphilic compounds to be used and the described formulation is for topical administration to the skin, not for ocular used. Moreover, a CBD oil is proposed for preparing the formulation, which does not mean necessarily that purified or isolated CBD, not being mixed with THC, is used, while in the present invention the micelle-encapsulated CBD is essentially free of THC, either because it has been isolated from *Cannabis sativa* plants or from one extract thereof or because it has been chemically synthesised in conditions where very low or no THC is also synthesised.

Thus, for the purposes of the present invention, the use of isolated CBD, or CBD chemically synthesised essentially lacking THC, is proposed. This also makes a difference with regard to some previous compositions for therapeutic use comprising CBD, such as the medicament commercialised in Canada and some European countries with the denomination Sativex^{™}, containing CBD mixed with THC. The use of isolated/pure CBD essentially free of THC in the present invention prevents the appearance of effects associated with this psychotropic substance. As used in the present invention, essentially free of THC means any possible THC present is at a concentration lower than 2% w/v, preferably lower than 1% w/v, more preferably below 0.2% w/v in the micelle solution.

With regard to the solvent, water, Type II water (which is the one used for the micelle solutions used in the Examples of the present application) can be used. Purified Type II water is preferred, particularly for those solutions intended to be administered (directly or after being added to a composition which comprises the micellar solution) to human beings. According to the American Society for Testing and Materials (ASTM: https://www.astm.org), Type I (also known as ultrapure) and Type II (also known as pure) are laboratory grade types of water which are defined as follows:
- Type I: water having a resistivity of >18 MΩ-cm, a conductivity of <0.056 µS/cm and <50 ppb of Total Organic Carbons (TOC)
- Type II: water having a resistivity of >1 MΩ-cm, a conductivity of <1 µS/cm and <50 ppb of TOCs.

As Type I is considered more appropriate for clinical, pharmaceutical or research uses such as advanced analytical methods (HPLC, etc.) or cell culture, it is preferred for the purposes of the present invention, particularly when the solution is prepared to be administered to a human being.

Any of the above mentioned water types can be prepared with any of the appropriate apparatus available in the market, such as those commercialised as Milli-Q^{™} water purification systems (MilliporeSigma^{™}, commercialised by Fisher Scientific in the United States and Europe).

The CBD encapsulated in the micelles formed by poloxamer 407 in water, preferably with the other ingredients of the above described composition being also present, and particularly when each of them is in the proportions used for obtaining the micellar solution used in the Examples of the present application, has shown to be suitable for topical ophthalmic administration of CBD in drops. Notwithstanding, the results indicate that not all CBD remains in the eye after administration, since CBD was administered only to one eye per mouse, and a therapeutic effect was observed in both eyes.

Although acute administration (one dose) resulted in increased IOP relative to the untreated group of glaucoma model animals (DBA/2J mice), repeated daily administration of CBD evoked a bi-phasic response in DBA/2J animals: an early (1 h-3 days. +70% - +35%) and a late (7 days on) phase. During the early phase, IOP was increased but repeated administration resulted in decreased values relative to the untreated group. Such a difference was more notorious after giving two doses (-80%) and gradually decreased soon after withdrawing treatment, but IOP was still reduced compared with the untreated group one week after finishing the treatment. These results make repeated administration preferable, that the administration takes place at least for more than one day, more preferably more than four days and even more preferably for more than seven days. Particularly because the additional parameters analysed do not make unadvisable such prolonged administration.

Repeated administration of CBD, for instance, also improved corneal mechanical sensitivity in DBA/2J. In this case, acute administration resulted in decreased mechanical sensitivity relative to the untreated group. Repeated daily administration of CBD kept mechanical sensitivity down relative to the untreated group (≃ -50% - -60%). Corneal mechanical sensitivity was still reduced one week after finishing the treatment.

Repeated administration of CBD had no effect on the spontaneous production of tears in DBA/2J. And, although a certain body weight decrease was observed during the phase of repeated CBD administration, larger in CD-1 mice, it was lower than 10% with regard to the weight before beginning the treatment, so that no severe affection of welfare is provoked by ocular topical CBD administration.

Thus, as commented above, chronic/repeated administration is preferred, which means that the micellar solution for use according to the present invention is to be administered, preferably, at least for more than one day, most preferably, at least for 4 days and even more preferably, at least for 7 days.

Given the biochemical and structural similarity of eyes among mammals (Fernandes et al., 2015), the micellar solution for use according to the invention can be administered to any mammal suffering from (or being susceptible to suffer from) glaucoma and/or IOP. Human beings are preferred.

The micellar solution for use according to the invention can be used as such, or may be an ingredient or component of a composition, preferably a pharmaceutical composition, for the same use. Also within the scope of the present invention are the compositions comprising the micellar solution for use according to the present invention, in all its possible embodiments, where one or more additional pharmaceutical acceptable excipient(s) is/are also present. The election of such additional excipients is an alternative possible within the knowledge and skills of one skilled in the art, depending on particular circumstances of, among others, the patient to treat, the intended administration regime or the place and/or conditions of storing of the composition, among others.

Although the Examples set forth below have been carried out with a micellar solution having the ingredients indicated in the formulation set forth in Example 1, in a particular concentration (CBD, poloxamer 407, sodium chloride, boric acid and sodium hyaluronate, all of them in type II water), the present invention is compatible with the concomitant administration, or the administration in the same treatment time period) of other drugs also used or proposed for the prevention and/or treatment of glaucoma and/or IOP. Thus, the composition for use according to the present invention can also comprise analogues of prostaglandins, β-adrenergic antagonists, cholinergic agonists, α2-adrenergic agonists, carbonic anhydrase inhibitors and/or rho-kinase inhibitors; the compositions for use according to the present invention, in all its possible embodiments, wherein one or more of those additional ingredients are is/also present, are also comprised within the scope of the present invention.

The invention will be now explained in more detail by the Examples below.

### EXAMPLES

### 1. Preparation and Properties of CBD-encapsulating micelles

Micelles and a micellar drop formulation were prepared and characterised. The following ingredients were used: CBD (Endoca BV, Hoofddorp, Netherlands); NaCl (Fisher Scientific S.L., Madrid, Spain); H₃BO₃ (Fisher Scientific S.L., Madrid, Spain); Low Molecular Weight Sodium Hyaluronate (LMW-HA: Contipro a.s., Doini Dobrou,č Czech Republic); Poloxamer 407 (purchased as Kolliphor^{®} P-407; BASF SE, Tarragona, Spain ); Type II Water (obtained with a Milli-Q^{™} IQ 7003 system; Fisher Scientific SL, Madrid, Spain).

### 1.1. Preparation of micelles and micellar solutions

The hydrophilic micellar ophthalmic drop formulation was prepared by i+Med, S.C. (Vitoria-Gasteiz, Spain). For that purpose, a micelle solution was first obtained with the following formulation:

| | |
|---|---|
| Poloxamer 407 | 4.5 g |
| CBD | 1.515 g |
| Absolute ethanol | 15 µl |
| Purified (type II) water | q.s.p. 50 ml |

Subsequently, the hydrophilic micellar drop formulation (the aqueous micellar solution to be administered as drops) was obtained by adding the 50 ml of micelle solution initially prepared to a solution with additional ingredients for the vehicle, to obtain a solution where the additional ingredients correspond to the following formulation:

| | |
|---|---|
| Boric Acid (H₃BO₃) | 7.50 g |
| NaCl | 0.075 g |
| LMW-HA | 0.625 g |
| Purified (type II) water | q.s.p. 500 ml |

The obtained formulation was maintained at 25°C until use, being the solution administered as ocular drops in the following Examples. It was received as a sterile, non cytotoxic and biocompatible solution with the characteristics indicated in Table 2 below:

**Table 2: Characteristics of the aqueous micellar solution**

| **Characteristic** | **Value** |
|---|---|
| Size of the micelles (Average diameter)^{a} | 30 - 60 nm |
| Viscosity^{b} | 29.66 cp |
| pH^{c} | 7.24 |
| % CBD (w/v)^{d} | 0.3 |
| Osmolality^{e} | 256.67 mOsmol/kg |
| Visual appearance^{f} | Transparent homogenous liquid |

| | |
|---|---|
| ^{a}Calculated from the Z-potential values; ^{b}Measured with a rotational viscosimeter (FungiLav/EvoExpert) following the method of the European Pharmacopoeia section 2.2.10. ^{c}Crison pH measurer, method of the European Pharmacopeia section 2.2.3; ^{d} percent weight/volume relation based on the total volume of the solution ^{e} Cryogenic osmometer Osmomat 3000 (Gonotec), method of the European Pharmacopeia section 2.2.35 ^{f} According to the refraction index obtained with an ABBE refractometer. | |

Asepsis of the manufacture procedure was confirmed through an assay of sterility of the micellar drop solution and the vehicle in accordance with ISO 11737-2 norm. No evidence of microbiological growth was observed.

The micelles of the micellar drop solution used in the following Examples of the present application are represented in Fig. 1, where it can be seen that CBD is encapsulated in the hydrophobic core of micelles.

### 1.2. Characterization of the micelles: CBD release rate

Next, an assay of CBD release from micelles through the HA hydrogel was carried out for 24 hours (the time that the gel will be in contact with the eye), using STF (simulated tear fluid: NaCl (0.68 g), NaHCO₃ (0.22 g), CaCl₂·2H₂O (0.008 g), KCI (0.14 g), and type II water to a total volume of 100 ml, filtered through a 0.2 µm pore size filter) as the receiving medium in order to recapitulate as much as possible release in tears, and taking samples at 1, 3, 5 8, 12 and 24 h. High-Performance Liquid Chromatography (HPLC) was the analytical technique that allowed the separation, identification (by comparison with standard patterns) and quantification, using a reverse phase and mobile phase under certain conditions; a mixture of methanol and water (85:15). The other conditions are as follows; flow rate of 1.0 ml/min, injection volume 10 µl and wavelength at 220 nm. Under the conditions mentioned above, a good determination of the CBD can be carried out.

The results are represented in Fig. 2, where it can be seen that the release is higher than 90% after 12 h. In particular, it is observed a gradual CBD release, higher and at a similar rate in the initial 6-8 hours, which begins to stabilise around 8-9 hours and which is slow but sustained approximately until 24 hours from the beginning of the assay. So, a stable and controlled release of CBD is observed over time after 24 hours.

### 2. Ocular administration and effects

In order to assess the feasibility and therapeutic efficacy of the topical ocular administration of the micellar solution of Example 1 or a composition comprising it, a research study in an animal model, mice, was designed and carried out.

### 2.1. Initial IOP study

In order to better design the research plan to carry out, an initial study was performed, where the evolution of lOP and other parameters with ageing was monitored.

The study was carried out with 40 DBA/2J mice during 10 months.

DBA/2J mice are double-knockout mice (Tyrpl^{b -/-} and Gpnmb^{R150X -/-}) which develop hereditary glaucoma associated with ageing. They undergo a chronic and extensive degenerative process characterised by eye abnormalities (iris atrophy, anterior synechia, pigment dispersion, high IOP, RGC degeneration), as well as collateral effects such as hearing loss, loss of cochlear nucleus neurons and alcohol and morphine intolerance. As it is known, symptoms begin around 3-4 months of age and become moderate between months 6-7. They exhibit elevated IOP of variable incidence at the age of 6-12 months (specifically at the age of 9 months (♂:16.2±1.4mmHg | $:20.3±1.8mmHg) in the assays described below). IOP may vary ± 3.5 mm Hg during a 24-hour cycle; IOP may vary with eyeball size. All general anaesthetics alter IOP in mice in a time-dependent manner. Measurements at different time intervals may yield different results (variations up to 8 mm Hg). IOP changes may be observed due to the effects of pulse, breathing, eye movements, and body position. The result will vary if the tip of the probe contacts different parts of the eye.

Mice were purchased at the age of 4 weeks and they underwent a phase of quarantine and acclimatisation. The study began when they were 16-week old and was carried out initially with 40 mice, although some of them were lost during the assays. IOP measures were taken with an iCare^{®} TA01i Tonolab Tonometer (iCare Finland Oy, Helsinki, Finland). Six measurements are needed to obtain the final reading. On each one of the days established for measurement, six final readings were taken, the highest and lowest results were excluded and the average of those 4 that favoured the general trend was calculated. The average value of the results obtained for each one of the observed eyes, as well as the days when measurements were taken, are indicated in Table 3.

**Table 3. IOP fluctuation of DBA/2J mice by age (measurements under anaesthesia)**

| Month | Age (weeks) | Eyes (n) | IOP (mm Hg) | | |
|---|---|---|---|---|---|
| | | | Min. | Max. | 95% C.I.* |
| 4 | 16 | 40 | 8 | 12 | 9.53 - 10.12 |
| | 19 | 40 | 7 | 11 | 8.83 - 9.42 |
| 5 | 21 | 40 | 7 | 18 | 10.39 - 12.26 |
| | 22 | 40 | 7 | 13 | 8.29 - 9.41 |
| 6 | 24 | 40 | 7 | 38 | 10.45 - 15.40 |
| | 25 | 38 | 7 | 39 | 12.10 - 17.69 |
| | 27 | 37 | 7 | 44 | 11.79 - 16.86 |
| 7 | 28 | 35 | 7 | 44 | 14.93 - 20.10 |
| 8 ^{b1} | 35 | 36 | 8 | 51 | 22.21 - 28.67 |
| 11 ^{b2} | 46 | 36 | 8 | 41 | 23.78 - 29.72^{a} |
| 12 | 49 | 36 | 7 | 36 | 14.04 - 18.68 |
| 13 | 55 | 34 | 7 | 34 | 10.82 - 15.83 |
| 14 | 59 | 33 | 7 | 36 | 12.40 - 17.60 |

| | | | | | |
|---|---|---|---|---|---|
| * 95% C.I. (confidence interval): ^{a} Conscious: 19.3 ± 0.4 mm Hg ^{b1,b2} Maximum peak | | | | | |

As the maximum peak was observed from month 8 to month 11, it was decided to design the plan of research of the effect of topical ophthalmic administration of CBD encapsulated in micelles of poloxamer 407 beginning the drug administration at month 8 and prolonging the study for more than one month, as explained in the following section.

### 2.2. Design of the research plan.

The study of the effects of topical ophthalmic CBD administration in micelles was carried out with outbred CD-1 mice and inbred DBA/2J mice. Male JAXTM DBA/2J mice homozygous for null mutations in Tyrplb and GpnmbR150X (-/-) were purchased from Charles River Laboratories (France). CD-1^{®} IGS male mice were obtained from the animal facilities of Universidad Rey Juan Carlos, using 51 mice in total for the study . All animals were 4 weeks-old at the beginning of the study. They were maintained in a temperature (23 ± 1 °C) and humidity (50-55%) controlled room, under a 12 h light/dark cycle (lights off between 19:00 and 07.00 h). Animals had free access to standard laboratory rat chow (Harlan Laboratories Inc.) and tap water. Trained investigators blind to drug treatment conducted all the behavioural assays in groups of two. Experiments were performed between 8:00 and 11:00 a.m. in conscious animals. All animal procedures were designed and conducted in accordance with a protocol approved by the Ethical Committee of Universidad Rey Juan Carlos and by the Autonomous Region of Madrid (PROEX 187.3/21) and followed the guidelines for the Care and Use of Laboratory Animals of the European Union Directive (2010/63/EU) and Spanish regulations (Law 32/2007, RD 53/2013 and order ECC/566/2015). A total of 51 male mice were utilised in this study, as explained in section 2.2 below. The number of animals used and their suffering were minimised

CD-1 mice are outbred mice, are vigorous, prolific and cheap to purchase, and have been widely used in research (Olson *et al.,* 2014), so that it can be considered equivalent to wild-type mice.

The research plan designed with the animal model is depicted in Fig. 3. It comprises three main phases:
- An ageing phase, which includes an initial 3-month period of quarantine and acclimatisation and an additional 4.5-month period during which IOP (from month 3 until month 7,5), tear production (from month 5,5 until month 7,5) and tactile sensitivity (from month 4 to month 7,5) are monitored to obtain a baseline for such magnitudes.
- A treatment phase, beginning when mice were 8 months old, where DBA/2J and CD-1 animals were randomly divided into two groups: treated and untreated. In this phase, treated mice receive CBD doses. The treatment phase comprises 3 periods: an acute period of 1 day, where, at time 0 of the phase, mice receive a CBD dose and are monitored for 24 hours; an initial chronic period of 18 days (days 2 to 19 of the treatment phase), where mice receive a daily CBD dose, and a second chronic period of other 18 days (days 21 to 37 of the treatment phase), where mice receive 2 daily CBD doses. From the end of the ageing phase to the end of the following phase, the wear-off phase, IOP, tear production and tactile sensitivity are monitored three times per week, following an alternative measuring system: on a same day, IOP was monitored in two groups, while tear production and tactile sensitivity were monitored in the other two groups, while, on the following measuring day, tear production and tactile sensitivity were monitored in the first two groups and IOP was monitored in the other two groups.
- A wear-off phase, during which mice receive no treatment and IOP tear production and tactile sensitivity are monitored at 24, 48, 72 and 96 hours after the beginning of the phase, following the same alternative system of the treatment phase.

The mice were distributed in cages as follows: cages J277 and J280 with 10 and 11 DBA/2J mice respectively, cages J278 and J279 with 10 DBA/2J mice each, and two additional cages with 5 CD-1 mice.

A more detailed version of the research plan, with the exact days of September, October and November where actions were taken, is represented in Table 3, where the information related to the groups of mice which received treatment (J277 and J280) is shaded, while the information related to the groups of mice untreated (J278 and J279) is not shaded.

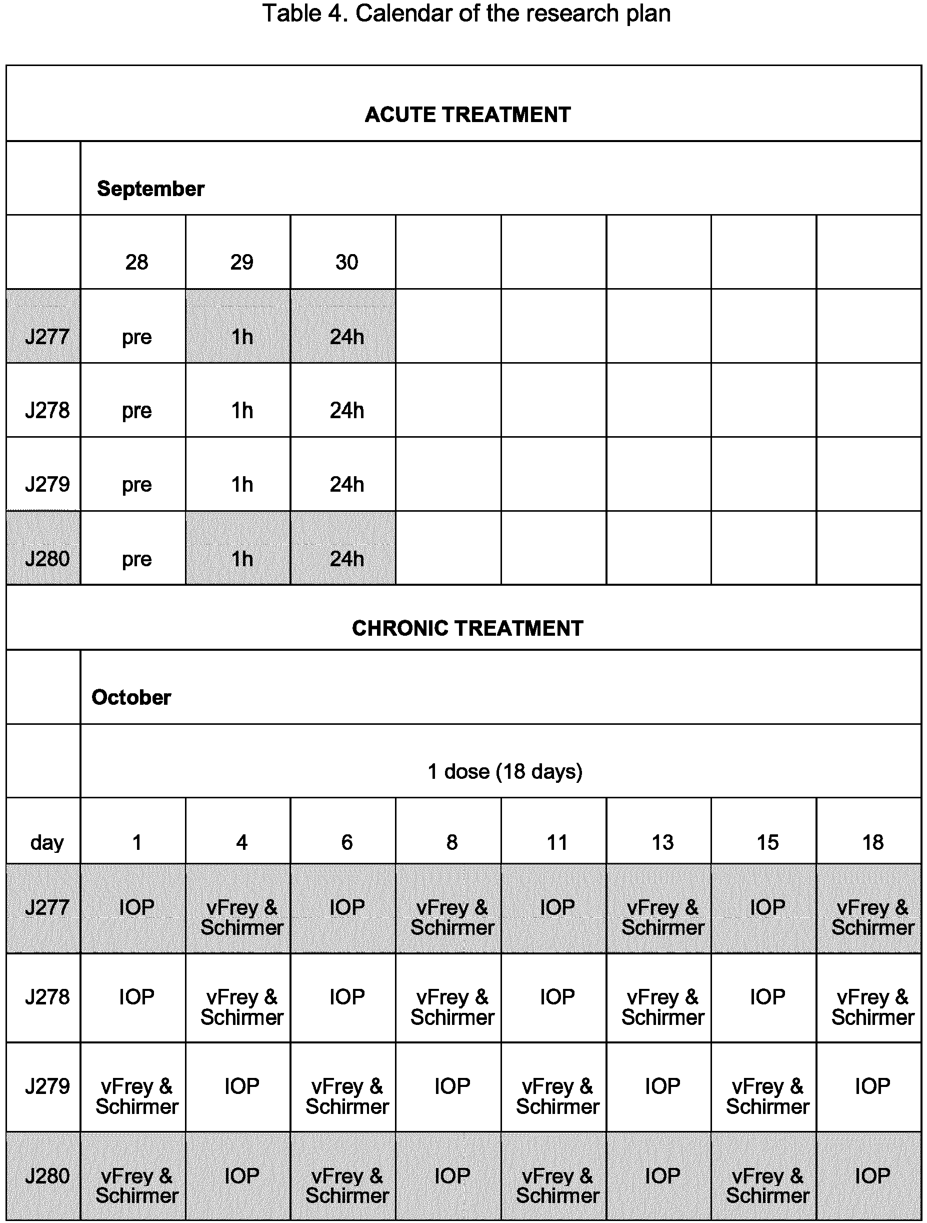

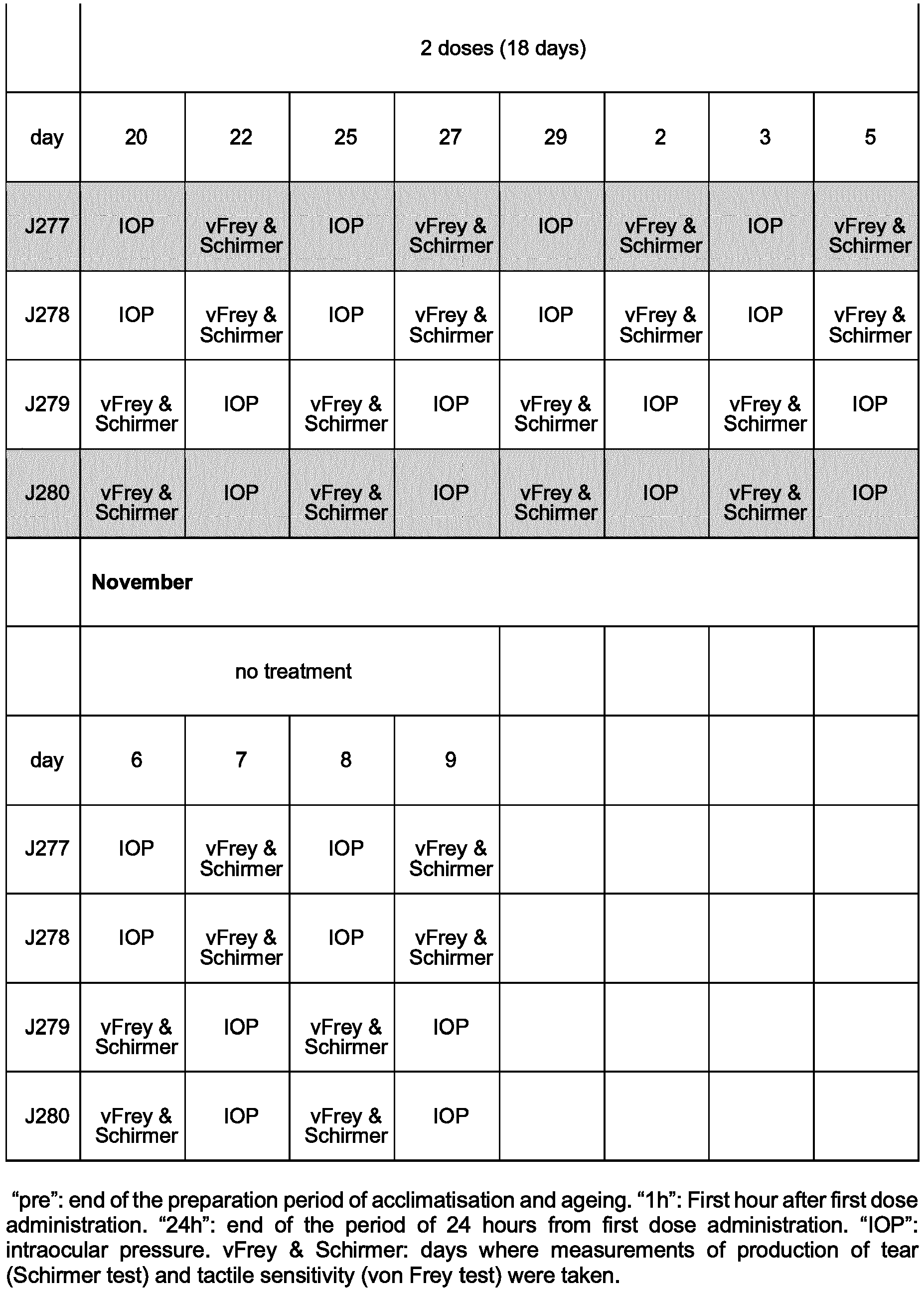

CBD was administered as part of a micellar solution prepared as described in Example 1. In particular, in each dose, 5 µl of the micellar solution (with 5 mM CBD) was administered to one eye of each mice, while the other eye remained untreated or received only the vehicle, type II water. Treatment was always applied to the right eye. A different group of animals was treated during two weeks with the vehicle to ensure that it had no effect on IOP, corneal mechanical sensitivity or spontaneous production of tear.

IOP, production of tear and tactile sensitivity were monitored as indicated in Fig. 3 and Table 3. Measurements during the chronic treatment phase were always taken prior to daily administration. At each time point indicated in the chart of Fig. 3, rebound tonometry or von Frey test was performed in the same group of animals. The Schirmer test was conducted after the Von Frey test. The time between the first and second dose, as required, was 8-10 hours.

All data were analysed and plotted on graphs using the GraphPad Prism 8.0 software. All data are expressed as mean ± standard error of the mean (S.E.M.). The normality of all data was assessed. One-way ANOVA, followed by Dunnett's multiple comparisons test, was used to compare the effect of drugs in relation to the absence of treatment. Two-way ANOVA (drug x dose) followed by Sidak's multiple comparisons test were used to determine statistical significance between left and right eyes. Values of P < 0.05 were considered statistically significant.

The measurement procedures and the obtained results are commented on in the following sections.

### 2.3. IOP study of treated and untreated mice

DBA/2J mice again were used in order to assess the influence of topical ophthalmic administration of CBD on IOP evolution in mice, as animal models of IOP increase with age and glaucoma development.

As explained above, mice were divided in four groups of 10 individuals each in cages as follows: groups J277 and J280, with 10 and 11 DBA/2J mice respectively; groups J278 and J279, with 10 DBA/2J mice each. Two groups of DBA/2J (J277 and J280) mice received CBD treatment, while the other two (J278 and J279) remained untreated.

For each group, IOP measurements were taken following the research plan of Fig. 3 and the specific calendar of Table 3. An iCare^{®} TA01i Tonolab Tonometer (iCare Finland OY, Finland) was used. For each eye, ipsilateral and contralateral measurements were taken. The number of measurements and the way of analysing them were as indicated in section 2.1. The results are represented in Fig. 4 and Fig. 5.

In Fig. 4, the evolution of IOP, in mmHg, over time can be seen for the different phases of the plan, both for untreated mice (panel A) and treated mice (panel B). Comparisons are made between contralateral and ipsilateral eyes. The results of performing two-way ANOVA followed by Sidak's test are represented in the graphs. This test is a statistical method used to counteract the problem of multiple comparisons by adjusting the p-values to control the family error rate, because this type of error increases as the number of comparisons does, as the individual tests are statistically independent. As can be seen, IOP evolution is identical for both eyes.

In Fig. 5, the evolution of IOP, in mmHg over time can be seen for the different phases of the plan, both for contralateral eyes (panel A) and ipsilateral eyes (B). In each graph. comparisons are made between untreated and treated groups. Again, the results of performing two-way ANOVA followed by Sidak's test are represented in the graphs. As can be seen from the graphs, in treated mice lOP shows an early increase during the first days after beginning of CBD treatment, followed by a decrease phase, while untreated mice show an IOP increase during the decrease phase of treated mice, excepting the last value in the treatment phase.

This can be also seen in the following Table, prepared with the ratios between the IOP values obtained at each measurement time (tₙ) and the IOP value at the beginning of the study (time 0, t₀= month 3.0) for each group (contralateral non-treated, ipsilateral non treated, contralateral treated, ipsilateral treated). The differences between the values obtained in the contralateral measurements (treated minus non treated) and the ipsilateral measurements (treated minus non treated), for each time point, are indicated in the Table as well.

| | **Ageing phase** | | | **Acute** | **Chronic** | | | | | | | | **Wear-off** | |
|---|---|---|---|---|---|---|---|---|---|---|---|---|---|---|
| | month | | | hour | Days | | | | | | | | | |
| | **3.5** | **5.5** | **7.5** | **1** | **3** | **7** | **12** | **16** | **21** | **26** | **30** | **35** | **38** | **40** |
| **contra- (non-treated)** | **22%** | **58%** | **90%** | **97%** | **51%** | **70%** | **98%** | **202%** | **160%** | **234%** | **232%** | **184%** | **108%** | **205%** |
| **ipsi- (non-treated)** | **17%** | **88%** | **106%** | **111%** | **95%** | **136%** | **193%** | **251%** | **171%** | **174%** | **227%** | **163%** | **159%** | **235%** |
| **contra- (treated)** | **22%** | **53%** | **90%** | **192%** | **131%** | **119%** | **128%** | **117%** | **100%** | **97%** | **113%** | **96%** | **64%** | **171%** |
| **ipsi- (treated)** | **13%** | **82%** | **99%** | **180%** | **130%** | **101%** | **104%** | **77%** | **90%** | **95%** | **68%** | **81%** | **81%** | **165%** |
| **Δ[ipsi-(treated)-ipsi-(non-treated)]** | **-4%** | **-6%** | **-7%** | **+69%** | **+35%** | **-35%** | **-89%** | **-174%** | **-81%** | **-79%** | **-159%** | **-82%** | **-78%** | **-70%** |
| **Δ[contra-(treated)-contra-(non-treated)]** | **0%** | **-5%** | **0%** | **+95%** | **+80%** | **+49%** | **+30%** | **-85%** | **-60%** | **-137%** | **-119%** | **-88%** | **-44%** | **-34%** |

Summarising, acute administration resulted in increased IOP relative to the untreated group. However, repeated daily administration of CBD evoked a bi-phasic response in DBA/2J animals: an early (1 h-3 days. +70% - +35%) and a late (7 days on) phase. During the early phase, IOP was increased but repeated administration resulted in decreased values relative to the untreated group. Such a difference was more notorious after giving two doses (-80%) and gradually decreased soon after withdrawing treatment, but IOP was still reduced compared with the untreated group one week after finishing the treatment.

### 2.4. Corneal mechanical sensitivity.

The study was carried out with the same DBA/2J animals used in the study of section 2.3, taking measures of tactile sensitivity in days different from those where IOP was measured, as scheduled in Fig. 3 and Table 3. Additionally, 10 CD-1 mice were included in this study, following the same research plan, and during the same time period than the DBA/2J ones: 5 of them were treated as the DBA/2J treated mice and the other 5 received only drops of vehicle (type II water).

Corneal mechanical sensitivity was measured following the von Frey test. The Von Frey test, also known as the filament test, is a method used to measure the sensitivity to touch or mechanical pressure of an animal or human. It is commonly used to assess pain sensitivity in animals. The test uses a series of thin filaments to apply pressure at a specific location, so the filament with the lowest force that produces a response is recorded as the threshold for that location (Deuis *et al.,* 2017). In the present study, measures were obtained using the following von Frey filaments: 0.008g, 0.02g, 0.04g, 0.07g, 0.1g (Aesthesio^{®}, Ugo Basile, Italy).

The results have been represented in Figs. 7, 8, 9 and 10. In Fig. 6 (DBA/2J mice) and Fig. 7 (CD-1 mice), which allow the comparison between contralateral and ipsilateral eyes in treated and untreated mice, it can be seen that corneal mechanical sensitivity is identical for both eyes. Fig. 9 shows no significant effect on corneal mechanical sensitivity for CD-1 mice. For DBA/2J mice, to the contrary, Fig. 8 shows how repeated administration of CBD also improved corneal mechanical sensitivity: acute administration resulted in decreased mechanical sensitivity relative to the untreated group, while repeated daily administration of CBD kept mechanical sensitivity down relative to the untreated group (≃ -50% - -60%), being still reduced one week after finishing the treatment.

### 2.5. Spontaneous production of tear.

The study was carried out with the same mice (DBA/2J and CD-1) used in the study of section 2.4, taking measures of tear production in the same days as those of corneal sensitivity, as scheduled in Fig. 3 and Table 3.

Their analysis was carried out following the Von Schirmmer test, which consisted of an ophthalmologic examination used to measure tear production in the eyes by inserting a small special piece of absorbent paper in the corner of the eye to measure the amount of tears produced in a determined period of time. In brief, 1 x 17-mm filter paper strips were cut and a 2.5-mm fold was made at one end. The strips were passed through a UV-chamber for disinfection prior to use. The awake animal was gently held by one examiner and the folded tip of the strip placed into the conjunctival sac for 15 seconds. Then, the area of the wet test paper was measured (length x width).

The results have been represented in Fig. 10, where it can be observed no significant effect on spontaneous production of tear for any of the two mouse strains.

### 2.6. Assessment of actual severity at the end of the procedure

In order to assess the actual severity of the treatment at the end of the procedure, the rates of mortality and morbidity were also recorded. Additionally, body weight measurements were affected during the study, as body weight is considered to be a sign of welfare.

The values obtained are represented in Fig. 11. Additionally, the rate with regard to the value at month 7.8 (where CBD has not been administered yet) was calculated by one-way ANOVA followed by Dunnett's Multiple Comparisons test, a statistical test used to compare multiple treatment groups with a control group. Ia applicable when multiple treatment groups are present and it is desired it determine if any of them are statistically different from the control group, is often used when it is assumed that the variance is equal among all groups The results were over 10% decrease in body weight is normally considered as a severe effect of welfare, and such a level of decrease was not found in the present study for none of the mice.

However, a total of four casualties were encountered on the side of DBA/2J mice during this study. Two mice were found dead in their cage at 3.5 and 8-month timepoints (that is, before receiving treatment). One mouse was found with cognitive dysfunction (apparently vestibular disorder, treatment day 3) -this strain is homozygous for a cadherin mutation (Cdh23ahl) which leads to rapid hearing loss; this may explain the frequent bites during the last month. Another mouse was found with a severe corneal ulcer (treatment day 14). Both were sacrificed.

Excepting said cases, in general, it could be considered that the welfare of treated mice was not severely affected.

### References

Aebersold A, Duff M, Sloan L, Song Z-H. Cannabidiol Signalling in the Eye and Its Potential as an Ocular Therapeutic Agent. Cell Physiol Biochem. 2021 May 14; 55(Suppl 5): 1-14. doi: 10.33594/000000371
Allison K, Patel D, Alabi O. Epidemiology of Glaucoma: The Past, Present, and Predictions for the Future. Cureus. 2020 Nov; 12(11): e11686. Published online 2020 Nov 24. doi: 10.7759/cureus.11686
Deuis Jennifer R., Dvorakova Lucie S., Vetter Irina. Methods Used to Evaluate Pain Behaviours in Rodents, Frontiers in Molecular Neuroscience, 2017, DOI=10.3389/fnmol.2017.00284
Durgun E G , Güngör S, Ozsoy Y. Micelles: Promising Ocular Drug Carriers for Anterior and Posterior Segment Diseases. J Ocul Pharmacol Ther. 2020; 36(6):323-341. https://doi.org/10.1089/jop.2019.0109
Fernandes KA, Harder JM, Williams PA, et al. Using genetic mouse models to gain insight into glaucoma: Past results and future possibilities. Exp Eye Res. 2015;141:42-56. doi:10.1016/j.exer.2015.06.019
Gaudana R, Ananthula HK, Parenly A, Mitra AK. Ocular drug delivery. AAPS J. 2010 Sep;12(3):348-60. doi: 10.1208/s12248-010-9183-3. Epub 2010 May 1, 20223
Green K, Wynn H, Bowman KA: A comparison of topical cannabinoids on intraocular pressure. Exp Eye Res 1978;27:239-246
Heijl A, Leske MC, Bengtsson B, Hyman L, Bengtsson B, Hussein M, Early Manifest Glaucoma Trial Group. Reduction of intraocular pressure and glaucoma progression: results from the Early Manifest Glaucoma Trial. Arch Ophthalmol. 2002 Oct;120(10):1268-79. doi: 10.1001/archopht.120.10.1268
Ibeas Bih C, Chen T, Nunn AV, Bazelot M, Dallas M, Whalley BJ: Molecular Targets of Cannabidiol in Neurological Disorders. Neurotherapeutics 2015;12:699-730
Iffland K, Grotenhermen F. An Update on Safety and Side Effects of Cannabidiol: A Review of Clinical Data and Relevant Animal Studies. Cannabis Cannabinoid Res. 2017 Jun 1;2(1):139-154. doi: 10.1089/can.2016.0034.
Lowin T, Tingting R, Zurmahr J, Classen T, Schneider M, Pongratz G. Cannabidiol (CBD): a killer for inflammatory rheumatoid arthritis synovial fibroblast. s. Cell Death & Disease volume 11, Article number: 714 (2020)
Mandal A, Bisht R, Rupenthal ID, Mitra AK. Polymeric micelles for ocular drug delivery: From structural frameworks to recent preclinical studies. J Control Release. 2017 Feb 28;248:96-116. doi: 10.1016/j.jconrel.2017.01.012. Epub 2017 Jan 11. PMID: 28087407; PMCID: PMC5319397.
Miller S, Daily L, Leishman E, Bradshaw H, Straiker A. Δ9-Tetrahydrocannabinol and Cannabidiol Differentially Regulate Intraocular Pressure. Invest Ophthalmol Vis Sci. 2018 Dec; 59(15): 5904-5911. doi: 10.1167/iovs. 18-24838
Novella S. Where are we with CBD. Science-Based Medicine Archived, September 30, 2020
Oh, S. G.; Shah, D. O.: "Micellar Lifetime: Its Relevance to Various Technological Processes". Journal of Dispersion Science and Technology.2007;15 (3): 297-316. doi:10.1080/01932699408943559.
Olson E., Delyth Graham, Chapter 5 - Animal Models in Pharmacogenomics, Handbook of Pharmacogenomics and Stratified Medicine, Academic Press, 2014, Pages 73-87, https://doi.org/10.1016/B978-0-12-386882-4.00005-0.
Schaiquevich P, Riva N, Maldonado C, Vázquez M, Cáceres-Guido P. Clinical pharmacology of cannabidiol in refractory epilepsy. Farm Hosp. 2020 Jun 30;44(5):222-229. doi: 10.7399/fh.11390.
Shelton B: CBD Oil May Worsen Glaucoma. American Academy of Ophthalmology, 2019. URL: https://www.aao.org/eye-health/news/cbd-oil-may-worsen-glaucoma.
Storgaard L, Tran TL, Freiberg JC, Hauser AS, Kolko M. Glaucoma Clinical Research: Trends in Treatment Strategies and Drug Development. Front. Med., 13 September 2021, Sec. Ophthalmology, https://doi.org/10.3389/fmed.2021.733080
Taskar P, Adelli G, Patil A, Lakhani P, Ashour E, Gul W, ElSohly M, Majumdar S. Analog derivatization of cannabidiol for improved ocular permeation. J Ocul Pharmacol Ther. 2019;35(5):301-310. doi: 10.1089/jop.2018.0141
Thapa D, Cairns EA, Szczesniak AM, Toguri JT, Caldwell MD, Kelly MEM: The Cannabinoids Delta(8) THC, CBD, and HU-308 Act via Distinct Receptors to Reduce Corneal Pain and Inflammation. Cannabis Cannabinoid Res 2018;3:11-20.
Tomida I, Azuara-Blanco A, House H, Flint M, Pertwee RG, Robson PJ. Effect of sublingual application of cannabinoids on intraocular pressure: a pilot study. J Glaucoma 2006 Oct;15(5):349-53. doi: 10.1097/01 .ijg.0000212260.04488.60.
Wang G, Nie Q, Zang C, Zhang B, Zhu Q, Luo G, Wang S. Self-Assembled Thermoresponsive Nanogels Prepared by Reverse Micelle Positive Micelle Method for Ophthalmic Delivery of Muscone, a Poorly Water-Soluble Drug. J Pharm Sci. 2016 Sep;105(9):2752-2759. doi: 10.1016/j.xphs.2016.02.014. Epub 2016 Mar 31. PMID: 27041413.

## Claims

1. An aqueous micellar solution comprising micelles formed by an assembled poloxamer,
**characterized in that** cannabidiol (CBD) is encapsulated in the core of the micelles,
for use in the prevention and/or treatment of glaucoma, elevated intraocular pressure, ocular dryness, ocular discomfort or pain and/or high mechanical eye sensitivity,
wherein the solution is to be administered by the topical ophthalmic route.

2. The micellar solution for use according to claim 1, wherein the poloxamer is poloxamer 407.

3. The micellar solution for use according to claim 1 or 2, wherein the relative proportion poloxamer: cannabidiol in the solution is 9 : 3±0.30 weight: weight.

4. The micellar solution for use according to any one of claims 1 to 3, wherein the micellar solution comprises at least:
| | |
|---|---|
| Cannabidiol | 0.00282 - 0.00318 g/ml |
| Poloxamer | 0.00846 - 0.00954 g/ml |
| Type II Water | q.s.p. the desired volume. |

5. The micellar solution for use according to any one of claims 1 to 4, wherein the micellar solution additionally comprises:
| | |
|---|---|
| NaCl | 0.141 - 0.159 mg/ml |
| H₃BO₃ | 14.10 - 15.90 mg/ml |
| Sodium hyaluronate | 1.175 - 1.325 mg/ml |
| Type II water | q.s.p. the desired volume. |

6. The micellar solution for use according to any one of claims 1 to 5, wherein the micelles have an average diameter of 30 - 60 nm.

7. The micellar solution for use according to any one of claims 1 to 6, wherein the solution is to be administered at least for more than one day.

8. The micellar solution for use according to claim 7, wherein the solution is to be administered at least for four days.

9. The micellar solution for use according to claim 8, wherein the solution is to be administered at least for seven days.

10. The micellar solution for use according to any one of the preceding claims, wherein the micellar solution is for daily administration.

11. The micellar solution for use according to any one of the preceding claims, wherein the micellar solution is for being administered twice a day.

12. The micellar solution for use according to any one of claims 1 to 11, wherein the micellar solution is for being administered to a mammal.

13. The micellar solution for use according to claim 12, wherein the micellar solution is for being administered to a human being.

14. A composition comprising the micellar solution for use according to any one of claims 1 to 13, which additionally comprises one or more pharmaceutically acceptable excipient(s).

15. The composition for use according to claim 13 or 14, which additionally comprises one or more compound(s) selected from the group of: a prostaglandin analogue, a β-adrenergic antagonist, a cholinergic agonist, an α2-adrenergic agonist, a carbonic anhydrase inhibitor and a rho-kinase inhibitor.
